(19) Europäisches Patentamt
European Patent Office
Office européen des brevets

(11) **EP 1 716 225 B1**

(12) **EUROPÄISCHE PATENTSCHRIFT**

(45) Veröffentlichungstag und Bekanntmachung des Hinweises auf die Patenterteilung:
**14.11.2007 Patentblatt 2007/46**

(21) Anmeldenummer: **05715356.1**

(22) Anmeldetag: **16.02.2005**

(51) Int Cl.:
*C12M 1/00* (2006.01)      *C12M 3/00* (2006.01)
*C12P 21/00* (2006.01)      *C12N 5/00* (2006.01)
*C07K 14/505* (2006.01)

(86) Internationale Anmeldenummer:
**PCT/EP2005/001575**

(87) Internationale Veröffentlichungsnummer:
**WO 2005/080545 (01.09.2005 Gazette 2005/35)**

(54) **VERFAHREN ZUR HERSTELLUNG VON REKOMBINANTEN PROTEINEN IN EUKARYONTISCHEN WIRTSZELLEN**

METHOD FOR THE PRODUCTION OF RECOMBINED PROTEINS IN EUKARYONTIC HOST CELLS

PROCEDE POUR PRODUIRE DES PROTEINES DE RECOMBINAISON DANS DES CELLULES HOTES EUCARYOTES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **17.02.2004 DE 102004007658**

(43) Veröffentlichungstag der Anmeldung:
**02.11.2006 Patentblatt 2006/44**

(73) Patentinhaber: **Bioceuticals Arzneimittel AG 61118 Bad Vilbel (DE)**

(72) Erfinder:
• **HAKEMEYER, Christian 49324 Melle (DE)**
• **LÜTKEMEYER, Dirk 33611 Bielefeld (DE)**
• **LEHMANN, Jürgen 33615 Bielefeld (DE)**

(74) Vertreter: **Neuefeind, Regina Maiwald Patentanwalts GmbH Elisenhof Elisenstrasse 3 80335 München (DE)**

(56) Entgegenhaltungen:
WO-A-97/33973      WO-A-99/28455
US-B1- 6 180 401

• **CRUZ HELDER J ET AL: "Metabolic shifts by nutrient manipulation in continuous cultures of BHK cells" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, Bd. 66, Nr. 2, 1999, Seiten 104-113, XP002211961 ISSN: 0006-3592**
• **BURNS S. ET AL.: BLOOD, Bd. 99, 2002, Seiten 4400-4405,**
• **JIXIAN, D. ET AL.: CHI. J. BIOTECH., Bd. 13, 1998, Seiten 247-252,**
• **YANG, BUTLER.: BIOTECHNOL. PROG., Bd. 16, 2000, Seiten 751-759,**
• **YANG, BUTLER.: BIOTECHNOL. PROG., Bd. 18, 2002, Seiten 129-138,**

**Beschreibung**

Die vorliegende Erfindung betrifft ein Verfahren zur Herstellung von rekombinanten Proteinen in einer eukaryontischen Wirtszelllinie, das dadurch gekennzeichnet ist, dass die Kultivierung der Wirtszellen unter kontinuierlicher Zufütterung von Nährstoffen erfolgt, wobei die Geschwindigkeit der Zufütterung in linearer Abhängigkeit von der Dauer der Zufütterung erhöht wird.

Die Herstellung von rekombinanten Proteinen in Wirtszellen hat in den vergangenen Jahren stark an Bedeutung gewonnen, weil dadurch die Herstellung von therapeutischen Proteinen in großem Maßstab und mit einer hohen biologischen Sicherheit ermöglicht wird. Zur Herstellung von rekombinanten Proteinen können sowohl Bakterien wie *E. coli* oder Hefen wie *Saccharomyces cerevisiae* als auch Zellen von höheren Eukaryonten wie Insekten oder Säugern verwendet werden.

Bakterien und Hefen haben den Vorteil, dass sie sich rasch vermehren und geringe Ansprüche an das Kulturmedium stellen. Ein großer Nachteil dieser Wirtsorganismen ist aber, dass sie nicht in der Lage sind, Proteine richtig zu glykosylieren und zu sialylieren. Da aber ein korrektes Glykosylierungsmuster für die Funktionalität mancher Proteine unbedingt erforderlich ist, sind häufig die in Bakterien bzw. Hefen hergestellten Proteine biologisch nicht wirksam und können nicht zur Therapie eingesetzt werden. Für die Aktivität mancher Proteine, wie etwa Erythropoietin oder Interferon-beta, ist außerdem der Sialylierungsgrad, also der Gehalt der mit dem Protein verknüpften Sialinsäuren, Ausschlag gebend. Dabei weisen im Allgemeinen die Proteine mit einem höheren Sialylierungsgrad eine höhere spezifische Aktivität auf.

Um glykosylierte und/oder sialylierte Proteine zu erhalten, ist es daher erforderlich, Proteine, deren Aktivität von ihrer Glykosylierung und/oder Sialylierung abhängig ist, wie etwa Erythropoietin, t-PA (Gewebsplasminogen-Aktivator) oder den Blutgerinnungsfaktor VIII, in tierischen Zellkulturen herzustellen. Bei der Produktion von Medikamenten in tierischen Zellen können aber die Produktionskosten, u.a. aufgrund der im Vergleich zur Kultivierung von Mikroorganismen komplizierteren Mediumszusammensetzung, sehr hoch sein. Daher gewinnt die Entwicklung effizienter Produktionsverfahren zunehmend an Bedeutung.

Grundsätzlich lassen sich drei verschiedene Verfahrensweisen für die Kultivierung von Wirtszellen zur Produktion von rekombinanten Proteinen unterscheiden.

Bei einem Batch-Prozess werden Medium und Zellen zu Beginn der Kultivierung in den Bioreaktor eingebracht. Bis zum Ende der Kultivierung werden weder Nährstoffe zugefügt noch Zellen aus dem Fermenter entfernt. Allein Sauerstoff wird zugeführt. Wenn eines oder mehrere Substrate verbraucht sind, wird der Prozess beendet und die Produkte werden geerntet. Eine Variation dieses Batch-Prozesses ist der "repeated Batch"-Prozess, bei dem am Ende der Fermentation ein Teil des Kulturvolumens als Inokulum im Bioreaktor gelassen und mit neuem Medium aufgefüllt wird.

Der größte Vorteil des Batch-Prozesses liegt in seiner einfachen Umsetzung. Nachteilig ist jedoch, dass im Allgemeinen die Kapazität der Zellen zur Herstellung der rekombinanten Proteine nicht vollständig ausgeschöpft wird, da nur eine geringe Zelldichte erreicht wird, wodurch auch die Produktkonzentration in der Regel niedrig ist. Ein weiterer Nachteil des Batch-Prozesses liegt im ungünstigen Verhältnis zwischen der Produktionszeit und der Gesamtzykluszeit, die sich aus der Zeit für den Aufbau, die Reinigung und die Sterilisation des Bioreaktors zusammensetzt.

Das zweite Kultivierungsverfahren ist das kontinuierliche Verfahren, bei dem ständig frisches Medium zugeführt und im gleichen Maße Fermenterinhalt entnommen wird. So kommt es zu einer ständigen Zufuhr von Nährstoffen, wobei gleichzeitig wachstumshemmende Substanzen wie Ammonium und Laktat verdünnt werden.

Daher können mit dieser Strategie hohe Zelldichten erreicht und über einen vergleichsweise langen Zeitraum aufrechterhalten werden. Ein Spezialfall der kontinuierlichen Prozessführung sind Dialysereaktoren, bei denen hochmolekulare Substanzen wie Proteine im Fermenter zurückgehalten werden, während niedermolekulare Substanzen wie Substrate zugefügt oder die Hauptabfallprodukte Ammonium und Laktat aus dem System entfernt werden können.

Die Nachteile der kontinuierlichen Prozessführung liegen zum einen im hohen apparativen Aufwand, zum anderen müssen große Mengen Medium steril bereitgestellt und große Mengen Kulturüberstand aufgearbeitet werden. Da der Fermenterinhalt ständig ausgetauscht wird, kann das Produkt, wenn kein Dialysereaktor verwendet wird, nicht zu hohen Konzentrationen akkumulieren, was die Aufarbeitung zusätzlich erschwert.

Das dritte mögliche Verfahren schließlich ist die Fed-Batch-Fermentation, bei der die Kultivierung mit einem Bruchteil des gesamten Fermentervolumens gestartet wird und sofort oder nach kurzer Anwachsphase frisches Substrat zugegeben wird. Dadurch werden höhere Zelldichten und längere Prozesszeiten als im Batch-Verfahren ermöglicht. Ein Vorteil dieses Verfahrens ist es, dass sich der Stoffwechsel der Zellen über die Steuerung der Zufütterung beeinflussen lässt, was zu einer geringeren Produktion von Abfallsubstanzen führen kann. Gegenüber dem kontinuierlichen Verfahren besteht der Vorteil darin, dass das Produkt der Zellen über einen längeren Zeitraum im Fermenter akkumulieren kann und somit höhere Produktkonzentrationen erreicht werden, wodurch die anschließende Aufarbeitung erleichtert wird. Allerdings setzt dies voraus, dass das Produkt der Zellen stabil genug ist, um bei mehrtägiger Verweilzeit im Fermenter nicht enzymatisch abgebaut zu werden. Ein Nachteil dieses Verfahrens ist, dass sich die physiologischen Verhältnisse im Bioreaktor durch die Zufütterung konzentrierter Substratlösungen stark verändern können.

Ein Hauptproblem bei der Kultivierung tierischer Zellen unabhängig vom gewählten Verfahren ist, die Zellen mit genügend Nährstoffen zu versorgen, ohne dass die Abbauprodukte der Nährstoffe über ein für die Zellphysiologie kritisches Level hinaus akkumulieren. Als Hauptenergiequellen von tierischen Zellen dienen Glukose und Glutamin, deren Hauptabbauprodukte, Laktat bzw. Ammonium, in höheren Konzentrationen das Wachstum und den Stoffwechsel der Zellen hemmen und zum Absterben der Zellen führen (Hassell et al. (1991) Applied Biochemistry and Biotechnology 30: 29-41). Daher ist es bei der Kultivierung tierischer Zellen erforderlich, bei ausreichender Nährstoffzufuhr die Akkumulation von Laktat und Ammonium zu verringern, um somit höhere Zelldichten und eine höhere Produktausbeute erreichen zu können.

Eine Möglichkeit, die Produktion von Abfallprodukten zu reduzieren, besteht in der kontrollierten Substratzugabe, die auch als "catabolic control" bezeichnet wird. Dabei wird die Abhängigkeit des Zellmetabolismus von den Konzentrationen der Nährstoffe ausgenutzt. Es konnte gezeigt werden, dass eine limitierende Zufütterung von Glutamin und Glukose zu einer deutlich verminderten Produktion von Ammonium und Laktat bei Hybridoma-Zellen führt (Ljunggren & Häggström (1994) Biotechnology and Bioengineering 44: 808-818).

In Fed-Batch-Kulturen, in denen die Glukosekonzentration nach Messung des Sauerstoffverbrauchs der Zellen durch kontrollierte Zugabe von konzentriertem Medium angepasst wurde, konnte nach einiger Zeit eine Veränderung des Zellstoffwechsels beobachtet werden, die auch als "metabolic shift" bezeichnet wird. Dieser "metabolic shift" wird ausgelöst durch die Limitierung der Glukose- und Glutaminkonzentration im Medium über mehrere Tage hinweg und führt dazu, dass weniger Nährstoffe von den Zellen aufgenommen und verstoffwechselt werden. In der Folge steigt der Gehalt von Glukose und Glutamin im Kulturmedium an, während die Produktion der Abfallstoffe Laktat und Ammonium wegen des verminderten Verbrauchs deutlich zurück geht (Zhou et al. (1995) Biotechnology and Bioengineering 46: 579-587). Der "metabolic shift" wurde nicht nur bei Hybridoma-Zellen, sondern auch bei Zelllinien wie SPO, HEK-293, BHK und CHO beobachtet.

Durch den "metabolic shift" können hohe Zelldichten von über $1 \times 10^7$ Zellen pro Milliliter und lange Prozesszeiten erreicht werden, da die Abfallprodukte Laktat und Ammonium nicht in Konzentrationen akkumulieren, die das Zellwachstum hemmen. Wichtig ist dabei aber, dass die Zufütterungslösung an den Bedarf der Zellen angepasst ist, um das Erreichen des "metabolic shift" zu gewährleisten und sowohl Erschöpfung als auch übermäßige Akkumulation bestimmter Nährstoffe und damit ein starkes Ansteigen der Osmolalität zu vermeiden (Xie und Wang, 1994, Biotechnology and Bioengineering, 43, 1175 - 1189 und 1996, Biotechnology and Bioengineering, 51, 725 - 729). Weiterhin ist es von Bedeutung, den Zellen genügend Glukose als Substrat für die Glykosylierung der rekombinanten Proteine zur Verfügung zu stellen.

Damit die Konzentrationen der zugeführten Nährstoffe an den Verbrauch der Zellen angepasst werden können, werden in der Regel komplizierte Verfahren eingesetzt, um den momentanen Verbrauch der Zellen zu bestimmen, damit die Zufütterungsgeschwindigkeit daran angepasst und eine bedarfsgerechte Zufütterung durchgeführt werden kann. Dazu gehören unter anderem die Messung der Glukosekonzentration durch Fließinjektionsanalyse oder die Messung des Sauerstoffverbrauchs der Zellen.

So offenbart etwa US 6,180,401 B 1 ein Zellkulturverfahren, bei dem die Glukosekonzentration kontinuierlich gemessen und im Kulturmedium durch Anpassung der Zufütterung abhängig von den Messwerten innerhalb eines bestimmten Bereichs gehalten wird. Auch in US 2002/0099183 A1 bestimmt die Glukosekonzentration die Zufütterungsrate von Glukose, wodurch die Glukosekonzentration im Kulturmedium in einem bestimmten Bereich gehalten wird.

Eine bedarfsgerechte Nährstoffzugabe, die abhängig von der Glutaminkonzentration im Kulturmedium ist, ist in EP-A-1 036 179 beschrieben.

WO 97/33973 offenbart ein Kultivierungsverfahren, bei dem die Produktion eines elektrisch geladenen Stoffwechselprodukts anhand der Leitfähigkeit des Mediums gemessen und die Zufütterungsrate entsprechend angepasst wird.

US 5,912,113 beschreibt ein Fermentationsverfahren für Mikroorganismen, bei dem immer dann zugefüttert wird, wenn die Kohlenstoffquelle im Medium verbraucht ist und dadurch ein erhöhter pH-Wert oder eine erhöhte Gelöstsauerstoffkonzentration im Medium gemessen wird.

Die vorstehend beschriebenen Verfahren sind jedoch sehr aufwendig und bergen ein zusätzliches Risiko für die Sterilität des Bioreaktors, da sie die Messung eines oder mehrerer Kulturparameter mit Hilfe geeigneter Messgeräte erfordern.

Aufgabe der vorliegenden Erfindung ist es daher, ein Verfahren zur Verfügung zu stellen, mit dem rekombinante Proteine in Wirtszellen in hoher Konzentration und mit einem hohen Glykosylierungs- und/oder Sialylierungsgrad hergestellt werden können, wobei die Akkumulation von Abfallstoffen und die damit verbundene Hemmung des Zellwachstums durch eine kontrollierte Nährstoffzugabe und das Erreichen des "metabolic shift" verhindert wird und wobei auf aufwendige Messverfahren des Zellstoffwechsels und eine bedarfsgerechte Zufütterung verzichtet wird.

Diese und andere Aufgaben werden durch den Gegenstand des unabhängigen Anspruchs gelöst. Bevorzugte Ausführungsformen der vorliegenden Erfindung sind in den abhängigen Ansprüchen definiert.

Die Aufgaben der vorliegenden Erfindung werden gelöst durch die Bereitstellung eines Verfahrens zur Herstellung von rekombinanten Proteinen in einer eukaryontischen Wirtszelllinie, bei dem die Kultivierung der Wirtszellen unter kontinuierlicher Zufütterung von Nährstoffen erfolgt und die Geschwindigkeit der Zufütterung in linearer Abhängigkeit von der Dauer der Zufütterung erhöht wird.

Es wurde nun überraschenderweise gefunden, dass durch die kontinuierliche Zufütterung von Nährstoffen in Abhängigkeit von der Dauer der Zufütterung die Wirtszellen den "metabolic shift" erreichen können und in diesem über einen längeren Zeitraum, d.h. von in der Regel mindestens 3, bevorzugt mehr als 4 und besonders bevorzugt mindestens 6 Tagen, verbleiben können. Dabei wird nicht die Zufuhr des Mediums an den Verbrauch der Zellen angepasst, sondern die Zellen werden so mit Nährstoffen versorgt, dass sie ihren Verbrauch an die limitierte Nährstoffversorgung anpassen. Im Unterschied zum Stand der Technik findet keine Anpassung der Mediumzufuhr anhand der jeweils herrschenden Substratkonzentrationen oder der Stoffwechselaktivität statt, sondern die Mediumzufuhr erfolgt lediglich in Abhängigkeit von der Dauer der Zufütterung. Durch dieses Verfahren können hohe Konzentrationen rekombinanter Proteine mit einem hohen Glykosylierungs- und/oder Sialylierungsgrad erzielt und gleichzeitig aufwendige Messverfahren vermieden werden.

Insbesondere erfolgt die Zufütterung nach der Funktion

$$F = F_0 + F_1 \times t,$$

wobei die Parameter $F_0$ und $F_1$ auf der Basis der einmalig gemessenen Verbrauchsraten der Zellen frei wählbar sind und F die Fließgeschwindigkeit der Zufütterung (in ml/h) und t die Dauer der Zufütterung (in h) darstellt.

Bei Verwendung dieser Zufütterungsfunktion kommt es weder zur Akkumulation noch zur Limitierung von anderen Aminosäuren als Glutamin, dessen Zufuhr, ebenso wie die von Glukose, limitiert wird. Die Produktion der Abfallstoffe Laktat und Ammonium verringert sich kontinuierlich und kommt schließlich zum Erliegen. Der "metabolic shift" wird erreicht, wodurch der Verbrauch von Glukose und Glutamin verringert wird.

Nachdem der "metabolic shift" erreicht wurde, steigen die Konzentrationen von Glukose und allen Aminosäuren im Kulturmedium weiter stark an, weil die Zufütterungsgeschwindigkeit gemäß oben angegebener Funktion weiter zunimmt, während die Aufnahme und der Verbrauch von Glukose und Glutamin verringert ist. Die hohen Nährstoffkonzentrationen, insbesondere der Glukose, führen zur gewünschten Glykosylierung und einem hohen Sialylierungsgrad des rekombinant in der Wirtszelllinie hergestellten Proteins. Der Stoffwechsel der Zellen verbleibt im Zustand des "metabolic shift", die Wachstumsgeschwindigkeit der Zellen ist geringer.

Durch diese Prozessführung können die Zellen über einen langen Zeitraum kultiviert werden, was zu einer hohen Zelldichte und einer hohen Produktausbeute führt. Durch die zeitabhängige Zufütterung kann eine aufwendige Regelung der Nährstoffkonzentration, die von zellspezifischen Parametern abhängig ist und die eine Gefährdung der Sterilität darstellt, vermieden werden.

In einer bevorzugten Ausführungsform findet die Kultivierung der Wirtszellen in einem Fed-Batch-Verfahren statt.

Ebenfalls bevorzugt wird zur Zufütterung ein anderes Medium verwendet als das Startmedium, wobei das Startmedium eine geringere Konzentration von Glukose und Glutamin aufweist als das zur Zufütterung verwendete Medium, was zu einer Limitierung der Glukose- und Glutaminversorgung führt. Besonders bevorzugt wird zur Zufütterung ein Zufütterungskonzentrat verwendet.

Ebenfalls bevorzugt beginnt die Zufütterung nicht zeitgleich mit der Inokulation, besonders bevorzugt beginnt die Zufütterung 16 bis 22 Stunden nach der Inokulation, am meisten bevorzugt beginnt die Zufütterung 17 bis 18 Stunden nach der Inokulation.

Besonders bevorzugt werden vor der Kultivierung der Wirtszellen im erfindungsgemäßen Verfahren ihre Verbrauchsraten der Nährstoffe im Verhältnis zur Glukose bestimmt und auf dieser Basis die dem Zufütterungskonzentrat zuzusetzende Menge der Mediumsbestandteile (außer Glukose und Glutamin) berechnet. Am meisten bevorzugt findet diese Bestimmung der Verbrauchsraten in einem Batch-Verfahren ohne Limitierung der Glukose- und Glutaminkonzentration statt.

Der Begriff "Wirtszelllinie" bezeichnet eine Zelllinie, die stabil transformiert ist mit mindestens einem heterologen Nukleinsäuremolekül, wobei dieses Nukleinsäuremolekül das Gen für das herzustellende rekombinante Protein trägt. Dieses Gen wird von der Zelle exprimiert, d.h. transkribiert und translatiert. Optional sekretiert die Wirtszelllinie das Genprodukt. Heterologe Nukleinsäuremoleküle, die die Expression von rekombinanten Proteinen in Wirtszelllinien ermöglichen, sind im Stand der Technik bekannt und können u.a. nach den in Sambrook and Russell, Molecular Cloning - A laboratory manual, 3. Auflage 2001 beschriebenen Methoden hergestellt werden. "Transformiert" bedeutet hier explizit nicht die Immortalisierung von Zellen durch Onkogene, onkogene Viren, o.ä. Ebenso ist es möglich, das in einer humanen Wirtszelllinie vorhandene, aber nicht exprimierte Gen für das herzustellende Protein zu aktivieren, was z.B. durch homologe Rekombination, wie etwa offenbart in WO 91/09955 und WO 93/09222, erfolgen kann.

Bei der Wirtszelllinie kann es sich um jede beliebige Säugerzelle handeln, bevorzugt handelt es sich um Primaten- oder Nagerzellen. Besonders bevorzugt werden vom Hamster abgeleitete Zellen wie CHO (chinese hamster ovary cells)- oder BHK (baby hamster kidney)-Zellen oder humane Zellen wie HeLa- oder HEK293-Zellen verwendet. Am meisten bevorzugt werden CHO-Zellen verwendet. Die Wirtszelllinie kann so modifiziert sein, dass sie defizient für ein Stoffwechselgen wie z.B. Dihydrofolatreduktase (DHFR) ist, so dass es nach dem Einführen dieses Stoffwechselgens zusammen mit der für das rekombinante Protein kodierenden Sequenz in die Zelle durch Ausüben eines Selektionsdrucks unter Ausnutzung der Eigenschaften des Stoffwechselgens zur Amplifikation beider Gene kommt. Dies hat eine Steigerung der Genexpression zur Folge.

Die Kultivierung der Zellen kann grundsätzlich auf beliebige Weise erfolgen. Bevorzugt ist jedoch die Kultivierung als Suspension, z.B. in Flaschen mit Rührer oder in Rollerflaschen, die zur Fed-batch-Kultivierung geeignet sind.

Weiterhin ist bevorzugt, dass die zur Kultivierung verwendeten Medien einen nur geringen Serumgehalt, z.B. maximal 1% (v/v), aufweisen; besonders bevorzugt sind die Medien serumfrei. Beispiele für geeignete Kulturmedien sind Basalmedien wie etwa RPMI 1640, DMEM, F12 oder eRDF, die miteinander gemischt werden können und je nach Bedarf der Zellen mit Zusätzen in der geeigneten Menge versetzt werden. Besonders bevorzugt ist die Verwendung von DMEM/F12 in einem Mischungsverhältnis von 1:1 1 als Kulturmedium. In der am meisten bevorzugten Ausführungsform wird die Konzentration der Phosphationen im Medium durch Zugabe von zusätzlichen Phosphatsalzen erhöht. Geeignete Phosphatsalze sind die in der Grundzusammensetzung enthaltenen Phosphatsalze.

Geeignete Mediumszusätze neben Glukose und Aminosäuren sind Chelatbildner wie Aurintricarboxylsäure (ATA), anorganische Salze wie phosphathaltige Salze, Polyamine und deren Vorstufen wie Putrescin, Hormone wie Insulin, Antioxidantien wie Ascorbinsäure und Vitamingemische, Lipidvorläufer wie Ethanolamin und zellschützende Substanzen wie Pluronic F68.

Mit dem Begriff "Nährstoffe" im Sinne der Erfindung werden die Stoffe bezeichnet, die zur Energiegewinnung und zum Aufbau der Zellsubstanz in der Zelle nötig sind. Dabei handelt es sich bevorzugt um mindestens ein Kohlenhydrat und mindestens eine Aminosäure und besonders bevorzugt um Glutamin und Glukose.

Unter dem Begriff "rekombinantes Protein" werden Proteine verstanden, die in der Wirtszelllinie von einem heterologen Nukleinsäuremolekül exprimiert, d.h. transkribiert und translatiert, werden. Bevorzugt sind die rekombinanten Proteine humanen Ursprungs. Ebenfalls bevorzugt handelt es sich bei diesen rekombinanten Proteinen um glykosylierte Proteine wie Lymphokine, Cytokine, Immunglobuline und Hormone, wie z.B. Erythropoietin, t-PA, Blutgerinnungsfaktoren, G-CSF, GM-CSF, Thrombopoietin, Interferone und Interleukine.

Unter dem Begriff "glykosyliertes Protein" werden Proteine verstanden, die einen oder mehrere Zuckerreste kovalent an Aminosäurereste im Protein gebunden haben. Die Kohlenhydrate können entweder an Asparagin (N-glykosidische Bindung) oder an Serin oder Threonin (O-glykosidische Bindung) gebunden sein. Dabei können über die N- bzw. O-glykosidische Bindung Oligosaccharide mit unterschiedlicher Zuckerzusammensetzung an das Protein gebunden werden. Zu den Kohlenhydraten, die häufig als Zuckerreste in glykosylierten Proteinen vorliegen, gehören Fucose, Galaktose, N-Acetylgalaktosamin und Mannose. Die gebundenen Kohlenhydratstrukturen können chromatographisch über HPAEC-PAD (High pH Anion Exchange Chromatography mit Pulsed Amperometric Detection) nach enzymatischer Abspaltung der Kohlenhydrate mit Glykosidase, wie etwa in EP 1 036 179 beschrieben, analysiert werden

Besonders bevorzugt handelt es sich bei den rekombinanten Proteinen, die nach dem erfindungsgemäßen Verfahren hergestellt werden, um Proteine, bei denen ein hoher Sialylierungsgrad mit einer hohen spezifischen Aktivität korreliert, da durch das erfindungsgemäße Verfahren insbesondere ein hoher Sialylierungsgrad erreicht werden kann, am meisten bevorzugt handelt es sich bei dem rekombinanten Protein um Erythropoietin.

Unter "Sialylierung" versteht man das Anheften von Sialinsäureresten, das häufig an terminalen Zuckerresten in Glykoproteinen statt findet. Sialinsäuren sind N-Acylderivate der Neuraminsäure wie etwa N-Acetylneuraminsäure oder N-Glykolylneuraminsäure.

Der Sialylierungsgrad ergibt sich aus dem Verhältnis des gemessenen Stoffmengenanteils der Sialinsäure zum theoretisch möglichen Stoffinengenanteil der Sialinsäure in der betreffenden Kohlenhydratstruktur. Proteinmoleküle mit gleicher oder sehr ähnlicher Aminosäuresequenz, aber unterschiedlichem Sialylierungsgrad und unterschiedlichen isoelektrischen Punkten in der isoelektrischen Fokussierung werden häufig auch als "Isoformen" bezeichnet.

Unter dem Begriff "hoher Sialylierungsgrad" im Sinne der vorliegenden Erfindung wird verstanden, dass die nach dem erfindungsgemäßen Verfahren hergestellten rekombinanten Proteine einen durchschnittlichen Sialylierungsgrad von mindestens 70%, bevorzugt mindestens 80%, besonders bevorzugt mindestens 85% und am meisten bevorzugt mindestens 90% aufweisen. Im Falle des Erythropoietins bedeutet ein Sialylierungsgrad von mindestens 70% beispielsweise, dass von maximal 14 möglichen terminalen Sialinsäurebindungsstellen in einem Erythropoietin-Molekül mindestens 10 Bindungsstellen mit Sialinsäure besetzt sind.

In einem Kulturmedium, das ein Gemisch von Isoformen enthält, beträgt der Anteil der Isoformen mit einem hohen Sialylierungsgrad, bezogen auf die Gesamtmenge des rekombinant hergestellten Proteins im Kulturmedium, mindestens 40%, bevorzugt mindestens 50%, besonders bevorzugt mindestens 60%, insbesondere bevorzugt mindestens 70% und am meisten bevorzugt mindestens 80%.

Der mittlere Sialylierungsgrad kann zum Beispiel chromatographisch durch HPAEC-PAD (High pH Anion Exchange Chromatography mit Pulsed Amperometric Detection) nach enzymatischer Abspaltung der Sialinsäuren mit Neuraminidase, wie etwa in EP 1 036 179 oder EP 1 037 921 beschrieben, bestimmt werden. Durch Kapillarzonenelektrophorese (CZE), die auf der Ladung des Proteins beruht, kann sowohl der mittlere Sialylierungsgrad wie auch der Anteil der Isoformen mit einem hohen Sialylierungsgrad an der Gesamtmenge des rekombinant hergestellten Proteins festgestellt werden (vgl. European Pharmacopoiea, 4. Auflage 2002, 01/2002:1316). Zum qualitativen Vergleich von Proben mit unterschiedlichem Sialylierungsgrad eignet sich auch die Isoelektrische Fokussierung, bei der die Bandenmuster stärker ins saure verschoben sind, wenn mehr Sialinsäurereste am Protein vorhanden sind (vgl. European Pharmacopoiea, 4. Auflage 2002, S. 73-75).

Unter "Kultivierung der Zellen" wird verstanden, dass die Zellen *in vitro* unter Bedingungen gehalten werden, die die Proliferation, einen normalen Stoffwechsel der Zellen und die Bildung des rekombinanten Proteins erlauben, d.h. dass die Zellen mit allen nötigen Nähr- und Baustoffen sowie Sauerstoff versorgt werden.

"Kulturüberstand" bezeichnet das Kulturmedium, das über einen bestimmten Zeitraum mit den Zellen in Kontakt stand und dann von den Zellen separiert wurde. Der Kulturüberstand enthält das von den Zellen produzierte, rekombinante Protein. Die Abtrennung der Zellen erfolgt mittels üblicher Separationstechniken wie z.B. Filtration, Zentrifugation.

Unter "Zufütterung" im Sinne der vorliegenden Erfindung wird verstanden, dass zu einer Ausgangsmenge an Medium (Startmedium) weiteres Medium bzw. ein Konzentrat zugegeben wird, wobei das weitere Medium bzw. Konzentrat nicht die gleiche Zusammensetzung haben muss wie das bereits vorhandene Medium (Startmedium).

Unter "kontinuierlicher Zufütterung" wird im Rahmen der vorliegenden Erfindung verstanden, dass zu einer Ausgangsmenge an Medium ohne Unterbrechung weiteres Medium zugegeben wird.

Der Begriff "Zufütterungskonzentrat" bezeichnet eine Nährstofflösung, die eine größere Menge an Nährstoffen enthält als das Startmedium und zur Zufütterung verwendet wird.

Unter "Inokulation" wird im Rahmen der vorliegenden Erfindung verstanden, dass eine bestimmte Zahl von Zellen, die nach dem erfindungsgemäßen Verfahren kultiviert werden sollen, in Kultivierungsbehältern mit Medium in Kontakt gebracht und zum Wachstum angeregt wird.

"Fed-Batch-Verfahren" im Sinne der vorliegenden Erfindung bezeichnet ein Kultivierungsverfahren, bei dem zu Beginn der Kultivierung eine geringe Menge Startmedium im Bioreaktor vorliegt und im Verlauf der Kultivierung weiteres Medium bzw. Konzentrat zugefüttert wird, ohne dass Medium entnommen wird.

Unter "Startmedium" im Sinne der vorliegenden Erfindung wird das Medium verstanden, in dem die Zellen in dem Fermenter inokuliert und kultiviert werden, bevor die Zufütterung beginnt. Dieses Startmedium enthält eine geringere Konzentration von Glukose und Glutamin als das Zufütterungsmedium bzw. - konzentrat, wodurch die Glukose- und Glutaminversorgung limitiert wird.

Das Startmedium enthält 5 bis 500 mg/l, bevorzugt 200 bis 400 mg/l und besonders bevorzugt 250 mg/l Glukose. Weiterhin enthält das Startmedium 5 bis 100 mg/l, bevorzugt 20 bis 80 mg/l und besonders bevorzugt 50 mg/l Glutamin.

Das Zufütterungsmedium bzw. -konzentrat enthält mindestens 5g/l, bevorzugt mindestens 10 g/l, besonders bevorzugt mindestens 20 g/l und am meisten bevorzugt mindestens 30 g/l Glukose. Weiterhin enthält das Zufütterungsmedium bzw. - konzentrat mindestens 1 g/l, bevorzugt mindestens 2 g/l und besonders bevorzugt mindestens 2,5 g/l Glutamin.

Unter "Verbrauchsraten der Nährstoffe" wird im Rahmen der vorliegenden Erfindung die Änderung der Konzentration der Nährstoffe im Kulturmedium in einem bestimmten Zeitintervall in Abhängigkeit von der Zelldichte verstanden.

Unter "Kulturmedium" im Sinne der vorliegenden Erfindung wird das Medium verstanden, das zu einem bestimmten Zeitpunkt im Bioreaktor vorhanden ist. Während der Zufütterung handelt es sich um eine Mischung aus Startmedium und Zufütterungsmedium bzw. -konzentrat, wogegen sich das Kulturmedium vor der Zufütterung ausschließlich aus dem Startmedium zusammensetzt.

"Batch-Verfahren" im Sinne der vorliegenden Erfindung bezeichnet ein Kultivierungsverfahren, bei dem während der Kultivierung weder Medium zugeführt noch entnommen wird.

Im Rahmen der Erfindung wird die Wirtszelllinie für mindestens sechs Tage kultiviert, bevorzugt für mehr als acht, besonders bevorzugt für mehr als zehn und am meisten bevorzugt für etwa zwölf Tage. Die Dauer der Kultivierung berechnet sich ausgehend von der Inokulation und ist abhängig von der Viabilität der Zellen. Der oben beschriebene "metabolic shift", durch den sich der Verbrauch von Glukose und Glutamin und die Bildung von Laktat und Ammonium verringert, findet dabei in der Regel nach 4 bis 8 Tagen, bevorzugt nach 5 bis 7 Tagen und besonders bevorzugt nach 6 Tagen, berechnet ausgehend von der Inokulation, statt.

Das Verfahren der vorliegenden Erfindung ermöglicht es, Zelldichten von mehr als $10^7$ Zellen pro Milliliter Kulturmedium zu erreichen, bevorzugt werden Zelldichten von mehr als $1,3 \times 10^7$ Zellen pro Milliliter Kulturmedium erreicht, und besonders bevorzugt werden Zelldichten von mehr als $1,6 \times 10^7$ Zellen pro Milliliter Kulturmedium erreicht.

Ein weiterer Vorteil des vorliegenden Verfahrens ist, dass hohe Konzentrationen des rekombinanten Proteins im Kulturmedium erreicht werden. Es wird eine Konzentration von mehr als 60 mg/l Kulturmedium, bevorzugt mehr als 80 mg/l Kulturmedium, besonders bevorzugt mehr als 100 mg/l Kulturmedium, und am meisten bevorzugt mehr als 140 mg/l Kulturmedium erreicht.

Nach etwa zwölf Tagen Kultivierung im Kulturmedium wird bevorzugt eine Konzentration des rekombinanten Proteins von 100 bis 160 mg/l Kulturmedium, besonders bevorzugt von 110 bis 150 mg/l Kulturmedium und am meisten bevorzugt von 120 bis 150 mg/l Kulturmedium erreicht.

Bezogen auf die Zellzahl im Kulturmedium beträgt die Ausbeute nach zwölf Tagen Kultivierung mindestens 0,5 $\mu$g pro $10^5$ Zellen, bevorzugt mindestens 0,7 $\mu$g pro $10^5$ Zellen, insbesondere bevorzugt mindestens 1,5 $\mu$g pro $10^5$ Zellen, besonders bevorzugt mindestens 2,5 $\mu$g pro $10^5$ Zellen und am meisten bevorzugt mindestens 3,5 $\mu$g pro $10^5$ Zellen. Es wird nach zwölf Tagen Kultivierung eine Ausbeute von 0,5 bis 4 $\mu$g pro $10^5$ Zellen erreicht, bevorzugt beträgt die Ausbeute 1 bis 3,5 $\mu$g pro $10^5$ Zellen und am meisten bevorzugt beträgt die Ausbeute 2 bis 3,5 $\mu$g pro $10^5$ Zellen.

Das rekombinante Protein mit einem hohen Sialylierungsgrad kann aus dem Kulturmedium aufgereinigt und von Proteinen mit geringerem Sialylierungsgrad getrennt werden, indem es einem oder mehreren Reinigungsschritten unterworfen wird. Geeignete Aufreinigungsverfahren für Erythropoietin sind etwa auch in EP 0 830 376, EP 0 228 452 und WO 03/045 995 beschrieben.

Die vorliegende Erfindung wird in den nachfolgenden Beispielen, die nur der Veranschaulichung der Erfindung dienen und in keiner Weise als Einschränkung zu verstehen sind, erläutert. Dabei befassen sich die Beispiele exemplarisch mit der Herstellung von Erythropoietin in CHO-Zellen.

Beschreibung der Abbildungen

Abb. 1:    Schematische Darstellung des Bioreaktors

        a) für den Batch-Betrieb
        b) für den Fed-Batch-Betrieb

Abb. 2:    Verhältnis der spezifischen Verbrauchsraten der Aminosäuren im Vergleich zur spezifischen Verbrauchsrate von Glukose bei der Batch-Kultivierung der Wirtszelllinie

Abb. 3:    Zeitlicher Verlauf der Lebend- und Gesamtzelldichte sowie des Sauerstoffstellsignals während der Kultivierung der Wirtszelllinie nach dem erfindungsgemäßen Verfahren

Abb. 4:    Zeitlicher Verlauf der Konzentrationen von Glukose, Laktat, Glutamin und Ammonium während der Kultivierung der Wirtszelllinie nach dem erfindungsgemäßen Verfahren

Abb. 5:    Zeitlicher Verlauf der Proteinkonzentration, -menge und -produktivität während der Kultivierung der Wirtszelllinie nach dem erfindungsgemäßen Verfahren

Abb. 6:    Isoelektrische Fokussierung des nach dem erfindungsgemäßen Verfahren hergestellten Glykoproteins Spur 1 zeigt den BRP-Standard (European Pharmacopoiea (2002), 4. Auflage), Spur 2 zeigt das nach dem erfindungsgemäßen Verfahren hergestellte und danach aufgereinigte Erythropoietin, die restlichen Spuren zeigen Eluate unterschiedlicher Aufreinigungsschritte.

Tabelle 1: Zusammensetzung des Mediums für die Batch-Kultivierung

| DMEM/F12 (ohne NaHCO$_3$) + | |
|---|---|
| NaHCO$_3$ | 3 g/l |
| Glukose | 900 mg/l |
| Glutamin | 292 mg/l |
| Prolin | 20 mg/l |
| Asparaginsäure | 20 mg/l |
| Asparagin | 20 mg/l |
| Leucin | 50 mg/l |

(fortgesetzt)

| DMEM/F12 (ohne NaHCO$_3$) + | |
|---|---|
| Isoleucin | 50 mg/l |
| Ascorbinsäure | 25 mg/l |
| Eisensulfat | 2 mg/l |
| Putrescin | 0,32 mg/l |
| Aurintricarboxylsäure | 1,06 mg/l |
| Pluronic F68 | 1 g/l |
| Insulin | 10 mg/l |

Tabelle 2: Zusammensetzung des Startmediums für die Kultivierung nach dem erfindungsgemäßen Verfahren

| DMEM/F12 (ohne NaHCO$_3$, Glutamin und Glucose) + | |
|---|---|
| NaHCO$_3$ | 3 g/l |
| Glukose | 250 mg/l |
| Glutamin | 50 mg/l |
| Prolin | 20 mg/l |
| Asparaginsäure | 20 mg/l |
| Asparagin | 20 mg/l |
| Leucin | 50 mg/l |
| Isoleucin | 50 mg/l |
| Ascorbinsäure | 25 mg/l |
| Eisensulfat | 2 mg/l |
| Putrescin | 0,32 mg/l |
| Aurintricarboxylsäure | 1,06 mg/l |
| Pluronic F68 | 1 g/l |
| Insulin | 10 mg/l |
| NaH$_2$PO$_4$ x H$_2$O | 124,2 mg/l |

Tabelle 3: Zusammensetzung des Zufütterungskonzentrats für die Kultivierung nach dem erfindungsgemäßen Verfahren

| DMEM/F12 (ohne NaHCO$_3$ und NaCl) (in doppelter Konzentration) + | |
|---|---|
| NaHCO$_3$ | 3 g/l |
| Glukose | 33,5 mg/l |
| Asparaginsäure | 146 mg/l |
| Asparagin | 2100 mg/l |
| Serin | 920 mg/l |
| Glutamin | 2700 mg/l |
| Histidin | 364 mg/l |
| Glycin | 22 mg/l |

(fortgesetzt)

| DMEM/F12 (ohne NaHCO₃ und NaCl) (in doppelter Konzentration) + | |
|---|---|
| Threonin | 313 mg/l |
| Arginin | 858 mg/l |
| Tyrosin | 335 mg/l |
| Methionin | 257 mg/l |
| Valin | 520 mg/l |
| Tryptophan | 494 mg/l |
| Phenylalanin | 64 mg/l |
| Isoleucin | 746 mg/l |
| Leucin | 552 mg/l |
| Lysin | 384 mg/l |
| Prolin | 1000 mg/l |
| Cystein | 1500 mg/l |
| Ascorbinsäure | 250 mg/l |
| Eisensulfat | 20 mg/l |
| Putrescin | 3,2 mg/l |
| Aurintricarboxylsäure | 10,6 mg/l |
| Pluronic F68 | 1 g/l |
| Insulin | 50 mg/l |
| NaH₂PO₄ x H₂O | 248,4 mg/l |

Beispiele

1. Bestimmung der Glukose- und Laktatkonzentration im zellfreien Kulturüberstand

Die gleichzeitige Messung der Glukose- und Laktatkonzentration im zellfreien Kulturüberstand erfolgte durch den Analyzer YSI 2700 S (Yellow Springs Instruments, Yellow Springs, USA). Das Messprinzip beruht auf dem enzymatischen Umsatz von Glukose und Laktat zu Glukono-$\delta$-lakton bzw. Pyruvat und Wasserstoffperoxid, das amperometrisch nachgewiesen wird. Aus der Probenkammer, in der 30 $\mu$l der Probe mit dem YSI-Puffer vermischt werden, zweigen zwei getrennte Messstrecken für Glukose und für Laktat ab. Jeweils drei Membranen trennen dabei die Probe von den Elektroden. Die erste Membran hält Partikel ab. An der zweiten Membran erfolgt die Oxidation von Glukose bzw. Laktat durch membranimmobilisierte Enzyme (Glukose- bzw. Laktatoxidase). Die dritte Membran mit einer Ausschlussgrenze von 200 Dalton lässt größere anodisch reduzierbare Substanzen als Wasserstoffperoxid nicht passieren. Das bei der Enzymreaktion entstehende $H_2O_2$ kann dagegen die Membran durchdringen und wird an der dahinter liegenden Platinanode oxidiert. Der im Vergleich mit einem Ag/AgCl-Bezugssystem gemessene Stromfluss ist proportional zur Glukosebzw. Laktatkonzentration. Die Messung im Gerät erfolgt automatisch und dauert ca. eine Minute.

2. Bestimmung der Konzentration von Aminosäuren im zellfreien Kulturüberstand

Die Bestimmung der Konzentration der Aminosäuren wurde mittels reversed-phase HPLC mit Vorsäulenderivatisierung durchgeführt (Lim, 1987). Im alkalischen Milieu reagiert die Aminogruppe der Aminosäuren mit einem Derivatisierungsreagenz (50 mg ortho-Phtalaldyhyd (OPA) in 1 ml Methanol + 100 $\mu$l 3-Mercaptopropionsäure + 9 ml Boratpuffer, pH 10,4) zu einem fluoreszenzfähigen Isoindolderivat, das bei einer Anregungswellenlänge von 340 nm und einer Emissionswellenlänge von 450 nm detektiert werden kann. Die Trennung der Aminosäurederivate erfolgte mit einer RP18-Säule (Kromasil C18, 5 $\mu$m, Länge: 150 mm, Durchmesser: 4 mm, VDS Optilab Chromatographietechnik GmbH, Montabaur) aufgrund von hydrophoben Wechselwirkungen. Die mobile Phase bestand aus einem binären Puffersystem mit einem Gradienten von polarem zu unpolarem Puffer (Büntemeyer (1988) Entwicklung eines Perfusionssystems zur kontinuierlichen Kultivierung tierischer Zellen in Suspension, Dissertation, Universität Hannover). Die Auswertung geschah über die Berechnung der Peakflächen durch einen Vergleich mit Aminosäurestandards und dem internen Standard.

Zur Proteinfällung wurden 50 μl des zellfreien Überstandes mit 50 μl einer 5%igen Perchlorsäure (versetzt mit 300 μM delta-Aminovaleriansäure als internem Standard) vermischt. Nach Abzentrifugieren wurden 50 μl des Überstands mit 100 μl Kaliumboratpuffer (0,4 M, pH 9,5) in ein Eppendorf-Reaktionsgefäß pipettiert und gevortext. Nach erneutem Abzentrifugieren wurden 20 μl des Überstands in ein HPLC-Probengefäß gefüllt und in den Autosampler der HPLC-Anlage (VDS Optilab Chromatographietechnik GmbH) gestellt.

Prolin kann als sekundäres Amin nicht mittels der oben beschriebenen Methode derivatisiert werden. Die Fluoreszenzmarkierung der zellfreien Probe erfolgte mit OPA und 7-Chlor-4-nitrobenzo-2-oxa-1,3-diazol (NBD). Dazu wurden 20 μl interner Standard mit 20 μl zellfreier Probe vermischt und 50 μl des OPA-Reagenz zugegeben. Nach 1 min wurden 100 μl NBD-Lösung (10 mg/ml in Methanol) zugefügt und die Probe für 3 min bei 80°C im Thermomixer inkubiert. 100 μl wurden in ein HPLC-Probengefäß pipettiert und in den Autosampler der HPLC-Anlage (VDS Optilab Chromatographietechnik GmbH) gestellt. Die Quantifizierung erfolgte mittels reversed-phase HPLC.

3. Bestimmung der Ammoniumkonzentration im zellfreien Kulturüberstand

Das Messprinzip der Ammoniumbestimmung beruht, ähnlich wie das der Aminosäureanalyse, auf der Derivatisierung des Ammoniums im alkalischen Milieu zu einem fluoreszenzfähigen Produkt. Bei hohen pH-Werten wird fast alles Ammonium zu Ammoniak deprotoniert, das mit ortho-Phthalaldyhyd (OPA) und Thioglykolsäure zu einem Isoindolderivat reagiert und fluorometrisch nachgewiesen werden kann.

20 μl des zellfreien Überstandes wurden dazu mit 1,3 ml Derivatisierungsreagenz (250 mg OPA + 500 mg Thioglycolat + 2 ml Methanol p.a. + 100 ml 0,4 M Na-Boratpuffer, pH 10,4) in einer Micro-Quarzküvette vermischt. Diese aufbereitete Probe wurde im Fluoreszenzspektrometer (RF-551, Shimadzu, Kyoto, Japan) bei 415 nm Anregungswellenlänge und 485 nm Emissionswellenlänge gemessen. Die maximale Emission wurde nach ca. 1 min erreicht. Die Auswertung geschah mittels Zweipunkt-Eichung mit einem Ammonium-Standard (100 mg/l).

4. Bestimmung der Zelldichte und Viabilität

Die Zellzahlbestimmung erfolgte mittels eines Hämocytometers (Neubauer-Zählkammer).

Die Bestimmung der Viabilität erfolgte mittels der Exklusionsmethode. Zu diesem Zweck wurde die Zellsuspension vor der Analyse im Mikroskop im Verhältnis 1:1 (bei höheren Zelldichten auch 1:3) mit einer Trypanblau-Lösung (Sigma, Deisenhofen) vermischt.

5. Bestimmung der Produktkonzentration im zellfreien Kulturüberstand

Die Bestimmung der Produktkonzentration sowie die Prüfung auf Reinheit erfolgte mittels Reversed-phase HPLC, wie sie im Stand der Technik (z.B. WO 99/28346 und WO 03/45995) beschrieben ist.

6. Batch-Kultivierung der Wirtszelllinie zur Bestimmung des Nährstoffverbrauchs

Als Wirtszelllinie wurde eine CHO-Zelllinie verwendet, die defizient für das Dihydrofolatreduktasegen ist und in Urlaub und Chasin, 1980, PNAS 77:4216-4220 beschrieben ist. Diese Zellen sind unter der Nummer ATCC CRL-9096 bei ATCC hinterlegt. Die Zellen wurden stabil transformiert mit einem Expressionsvektor, der die Expression von humanem Erythropoietin unter der Kontrolle des CMV-Promotors steuert.

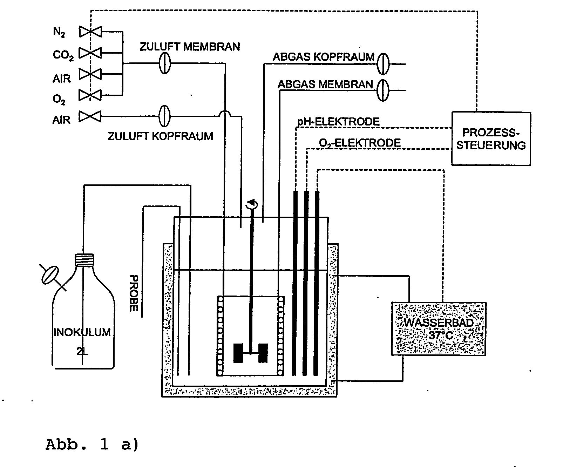

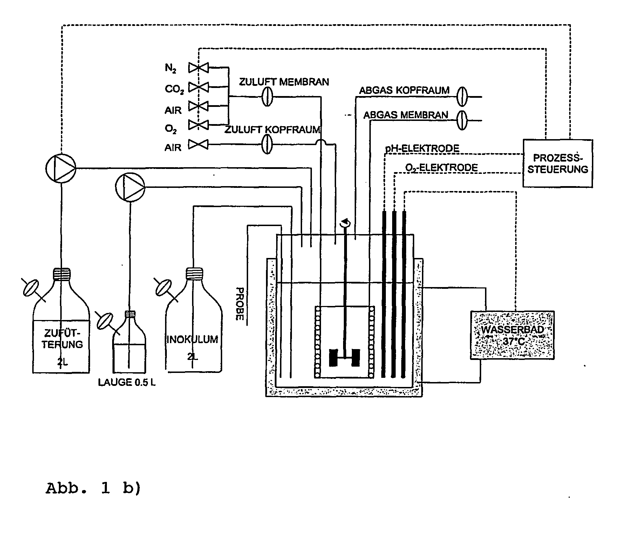

Zunächst wurden diese Zellen in einem Bioreaktor für die Batch-Kultivierung, wie in Abb. 1a) dargestellt, bei einer Temperatur von 37°C, einem Sauerstoffgehalt von 40% Luftsättigung und einem pH von 7,2 kultiviert.

Das zur Kultivierung verwendete Medium basierte auf einem 1:1 Gemisch von DMEM und F12 (Invitrogen, Karlsruhe) mit einer erhöhten Konzentration an einigen Aminosäuren und verschiedenen Zusätzen, die in Tabelle 1 aufgeführt sind, und einer nichtlimitierenden Konzentration von Glukose und Glutamin. Die Zellen wurden in einem Volumen von 21 kultiviert. Zur Inokulation wurden die Zellen während der exponentiellen Wachstumsphase aus einer Vorkultur entnommen. Die Inokulationszelldichte betrug etwa $2 \times 10^5$ Zellen pro Milliliter. Eine Kultivierung wurde abgebrochen, wenn der Sauerstoffgehalt im Medium nicht mehr durch externe Sauerstoffzufuhr auf einen Sollwert angepasst werden konnte und dadurch eine Limitierung der Kultur und ein Ende der Wachstumsphase angezeigt wurde. Nach Abschluss der Kultivierung wurde die Konzentration von Glukose, Laktat, Ammonium und Aminosäuren im Kulturmedium bestimmt (vgl. Beispiele 1-3).

7. Bestimmung des Nährstoffverbrauchs zur Herstellung des Zufütterungskonzentrats

Zur Berechnung der Aminosäurezusammensetzung des Zufütterungskonzentrats wurden die spezifischen Substratverbrauchsraten für die verschiedenen Aminosäuren während der Wachstumsphase bei der Kultivierung im Batch-Verfahren berechnet. Die spezifische Substratverbrauchsrate berechnet sich im Batch-Prozess aus der folgenden Gleichung:

$$qs = -\frac{S_1 - S_0}{t_1 - t_0} \cdot \frac{2}{X_0 + X_1}$$

Dabei ist t die Kulturdauer, x die Zelldichte, s die Konzentration des Substrats, und qs wird als spezifische Substratverbrauchsrate bezeichnet. Das Suffix 1 bezeichnet einen Wert zu einem bestimmten Zeitpunkt während der Kultivierung, das Suffix 0 bezeichnet den Wert beim Ausgangszeitpunkt.

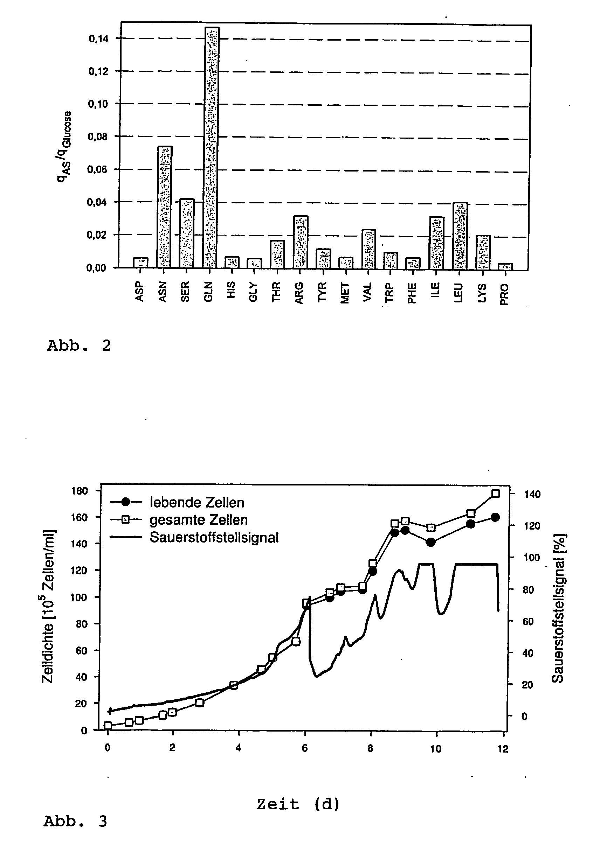

Anschließend teilt man die molaren Verbrauchsraten der Aminosäuren durch die molare Verbrauchsrate von Glukose, wodurch man ein Verhältnis erhält, welches angibt, wie viel Mol der jeweiligen Aminosäure pro Mol Glukose von den Zellen aufgenommen wurden (vgl. Abb. 2). Die Einwaage der Aminosäuren für die Zufütterungskonzentrate der Fed-Batch-Fermentationen orientierte sich an diesem Verbrauchsprofil aus den Batch-Kultivierungen und ist in Tabelle 3 dargestellt.

8. Kultivierung der Zellen und Herstellung des rekombinanten Proteins mittels des erfmdungsgemäßen Verfahrens in der Fed-Batch-Methode

Für die Fed-Batch-Kultivierung wurde ein Bioreaktor verwendet, wie er in Abb. 1 b) dargestellt ist. Zusätzlich wurde ein Abluftkühler (B. Braun) in den Deckel des Bioreaktors eingebaut. Die Kühlung erfolgt durch den Anschluss eines externen Kryostaten (Julabo Labortechnik GmbH, Seelbach), dessen Wassertemperatur auf 4°C eingestellt wurde. Eine Peristaltikpumpe (Schlauchdosierpumpe Modulsystem 1000, Petrogas KG, Berlin) ermöglichte die kontrollierte, zeitabhängige Zugabe des Zufütterungskonzentrates. Die Pumpe wurde durch ein Analogsignal mit einer Spannung zwischen 0 V und 10 V vom Computer gesteuert. Das zugehörige Programm ermöglichte die in Gleichung 1 dargestellte Zufütterungsfunktion.

$$\text{Gleichung 1: } F = F_0 + F_1 \times t$$

$F_0$ und $F_1$ sind frei wählbare Parameter, t die Zeit in Stunden seit Aktivieren der Zufütterung und F die Flussrate in Milliliter pro Stunde. $F_0$ wurde so gewählt, dass zu Beginn der Zufütterung der Verbrauch gedeckt wurde. $F_1$ wurde so gewählt, dass sich die Fließrate der Zufütterung nach 24 Stunden nach Beginn der Zufütterung verdoppelt hatte.

Die Fed-Batch-Kultivierung wurde jeweils mit einem Startvolumen von ca. 1.000 ml und einer Zelldichte zwischen $2 \times 10^5$ und $4 \times 10^5$ Zellen pro Milliliter aus den Vorkulturen gestartet. Im Gegensatz zur Batch-Kultivierung wurden die Zellen vor der Inokulation für fünf Minuten bei 800 rpm abzentrifugiert (Megafuge 1.0, Heraeus, Osterode) und dann in frischem Fed-Batch-Startmedium resuspendiert. Nach einer kurzen Anwachsphase (in der Regel zwischen 16 und 22 Stunden) im Startmedium wurde die Zufütterung mit dem Zufütterungskonzentrat gestartet, wobei die anfängliche Zufütterungsgeschwindigkeit meist dem aktuellen Verbrauch (bestimmt wie unter Beispiel 7 beschrieben) entsprach und sich innerhalb eines Tages verdoppeln sollte. Die Fermentationen wurden beendet, wenn die Viabilität der Zellen unter 90% sank.

Das Startmedium für die Fed-Batch-Kultivierung entsprach dem Medium für die Batch-Kultivierungen, allerdings enthielt es eine verringerte Glukose- und Glutaminkonzentration. Daher wurde zur Herstellung Glukose- und Glutamin-freies DMEM/F12-Medium verwendet (Sonderanfertigung, Applichem, Darmstadt), dem geringe Mengen dieser beiden Substrate zugesetzt wurden. Die Glukosekonzentration betrug im Startmedium daher nur 250 mg/l, die Glutaminkonzentration 50 mg/l. Zur Zusammensetzung des Startmediums vgl. Tabelle 2.

Zur Zufütterung wurde ein Medium verwendet, das bezogen auf die Glukosemenge zehnfach gegenüber dem normalen Medium konzentriert ist und deshalb als Zehnfach-Zufütterungskonzentrat bezeichnet wurde. Zur Herstellung dieses Zufütterungskonzentrats wurde NaCl-freies DMEM/F12-Medium (Sonderanfertigung, Applichem, Darmstadt) in doppelter Konzentration gelöst. Die Endkonzentration von Glukose, die sich zusammensetzt aus der im bezogenen DMEM/F12-Medium enthaltenen und der zusätzlich eingewogenen Glukose, betrug 40 g/l. Die Aminosäuren wurden entsprechend des vorher bestimmten Verbrauchs der Zellen jeweils im Verhältnis zur Glukose eingewogen. Andere Zusätze, wie Eisensulfat, Ethanolamin, Putrescin und Aurintricarboxylsäure wurden ebenfalls entsprechend des Konzentrationsfaktors des Mediums eingewogen. Die Zusammensetzung des Mediums ist in Tabelle 3 dargestellt.

Um die Zufütterungskonzentrate vollständig lösen zu können, wurde zunächst eine entsprechende Menge Reinstwasser (ca. 80% des Endvolumens) vorgelegt, und mit 1 M HCl auf einen pH-Wert kleiner pH 3 eingestellt. Unter Rühren wurden schrittweise die Aminosäuren, danach das Kochsalz-freie DMEM/F12-Medium und zum Schluss die übrigen Zusätze zugefügt. Um alle sauren Aminosäuren vollständig aufzulösen, wurde danach der pH-Wert mit 1 M NaOH auf pH 10,5 erhöht. Der dabei ausfallende, weiße Niederschlag löste sich bei der anschließenden Einstellung des pH-Wertes auf pH 7,5 wieder. Zuletzt erfolgte das Auffüllen auf das gewünschte Endvolumen mit Reinstwasser. Zusätzlich erfolgte die Zugabe einer Vitaminlösung (MEM-Vitamine, Biochrom KG, Berlin), so dass die Konzentration der zugegebenen Vitamine gegenüber dem Standardmedium fünffach erhöht war.

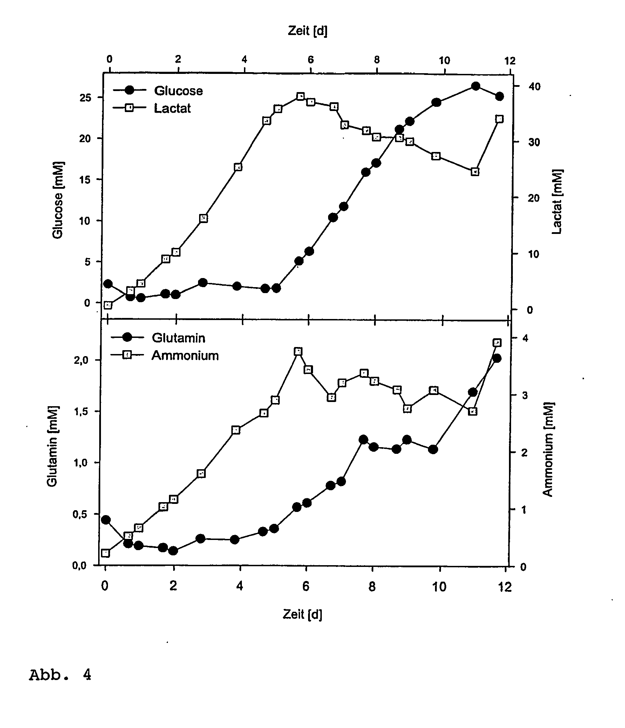

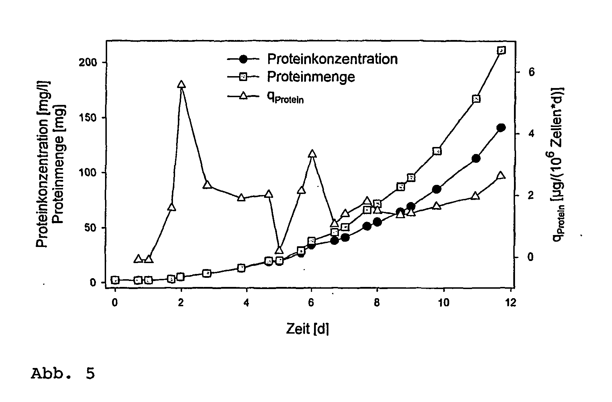

Die Zufütterung begann 17 Stunden nach der Inokulation. Die Parameter betrugen zunächst $F_0$ = 0,4 l/h, und $F_1$ = 0,022 ml/h$^2$. Um eine Überfütterung der Zellen zu vermeiden, wurde der Parameter $F_0$ jedoch 2,1 Tage nach Zufütterungsstart auf einen Wert von 0 ml/h korrigiert. Über den gesamten Verlauf der Kultivierung (12 Tage) wurde(n) die Lebend- und Gesamtzelldichte, das Sauerstoffstellsignal (nicht normiertes Stellsignal für das Sauerstoff-Massflowmeter), die Konzentrationen von Glukose, Laktat, Glutamin, Ammonium und rekombinantem Protein bestimmt, aber nicht dazu verwendet, die Zufütterung an den Nährstoffverbrauch anzupassen. Die Ergebnisse dieser Messungen sind in den Abbildungen 3- 5 dargestellt.

Die Abbildungen zeigen, dass bei der zeitabhängigen Zufütterung des Mediums der "metabolic shift" etwa sechs Tage nach der Inokulation erreicht wird, da die Konzentration von Ammonium und Laktat zu diesem Zeitpunkt nicht mehr weiter steigt, sondern vielmehr abnimmt. Die Konzentration des rekombinanten Proteins und die Zahl der lebenden Zellen nimmt jedoch über den gesamten Verlauf der Kultivierung zu.

9. Isoelektrische Fokussierung

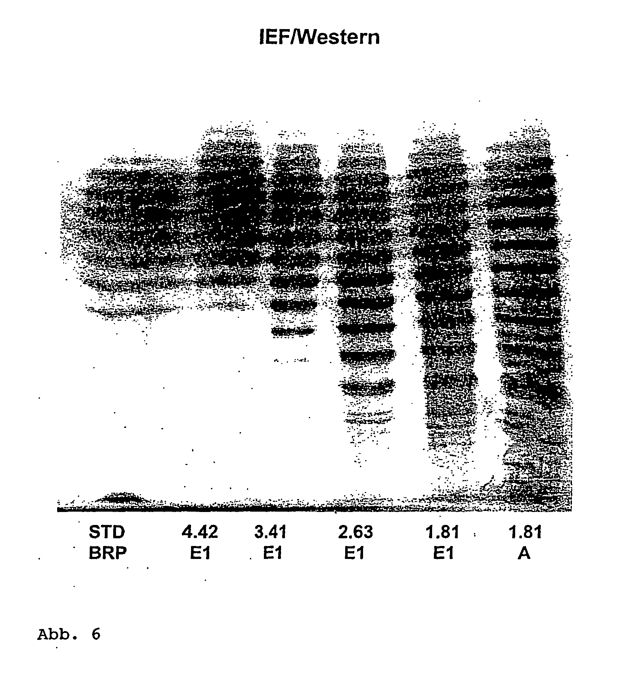

Die isoelektrische Fokussierung wurde durchgeführt wie in European Pharmacopoiea, 4. Auflage 2002, S. 73ff beschrieben, nachdem das Kulturmedium nach Verfahren, die im Stand der Technik beschrieben sind, aufgereinigt worden war. Das Ergebnis der isoelektrischen Fokussierung ist in Abbildung 6 dargestellt.

10. Kapillarzonenelektrophorese (CZE)

Die Kapillarzonenelektrophorese zur Bestimmung des Anteils des Erythropoietins mit hohem Sialylierungsgrad am Gesamt-Erythropoietingehalt wurde durchgeführt wie in European Pharmacopoiea, 4. Auflage 2002, 01/2002:1316 beschrieben.

**Patentansprüche**

1. Verfahren zur Herstellung von rekombinanten Proteinen in einer eukaryontischen Wirtszelllinie, wobei die Kultivierung der Wirtszellen unter kontinuierlicher Zufütterung von Nährstoffen erfolgt, **dadurch gekennzeichnet, dass** die Fließgeschwindigkeit der Zufütterung in linearer Abhängigkeit von der Dauer der Zufütterung erhöht wird.

2. Verfahren nach Anspruch 1, wobei es sich um ein Fed-Batch-Verfahren handelt.

3. Verfahren nach Anspruch 1 oder 2, wobei bei der Zufütterung ein anderes Medium verwendet wird als das Startmedium, wobei das Startmedium eine geringere Konzentration von Glukose und Glutamin aufweist als das zur Zufütterung verwendete Medium.

4. Verfahren nach Anspruch 3, wobei für die Zufütterung ein Zufütterungskonzentrat verwendet wird.

5. Verfahren nach einem der Ansprüche 1 bis 4, wobei die Zufütterung nicht zeitgleich mit der Inokulation beginnt.

6. Verfahren nach einem der Ansprüche 1 bis 5, wobei vor der Kultivierung der Zellen die Verbrauchsraten der Nährstoffe bestimmt werden.

7. Verfahren nach Anspruch 6, bei dem die Bestimmung der Verbrauchsraten in einem Batch-Verfahren erfolgt.

8. Verfahren nach einem der Ansprüche 1 bis 7, wobei während der Kultivierung die Verbrauchsraten der Nährstoffe nicht bestimmt werden.

9. Verfahren nach einem der Ansprüche 1 bis 8, wobei es sich bei den Zellen um Säugerzellen handelt.

10. Verfahren nach Anspruch 9, wobei es sich bei den Säugerzellen um Primaten- oder Nagerzellen handelt.

11. Verfahren nach Anspruch 9 oder 10, wobei es sich bei den Zellen um CHO-Zellen handelt.

12. Verfahren nach einem der Ansprüche 1 bis 11, wobei die Wirtszellen über einen Zeitraum von mehr als sechs Tagen kultiviert werden.

13. Verfahren nach einem der Ansprüche 1 bis 12, wobei zum Ende der Kultivierung die Zelldichte $\geq 10^7$ Zellen pro ml Kulturmedium ist.

14. Verfahren nach einem der Ansprüche 1 bis 13, wobei eine Konzentration an rekombinantem Protein von $\geq 60mg/l$ Kulturmedium erreicht wird.

15. Verfahren nach einem der Ansprüche 1 bis 14, wobei es sich bei dem rekombinanten Protein um ein glykosyliertes Protein handelt.

16. Verfahren nach Anspruch 15, wobei das rekombinante Protein einen hohen Sialylierungsgrad aufweist.

17. Verfahren nach Anspruch 15 oder 16, wobei es sich bei dem rekombinanten Protein um Erythropoietin handelt.

## Claims

1. Process for the production of recombinant proteins in a eukaryotic host cell line, wherein the culturing of the host cells is carried out with continuous feeding of nutrients,
   **characterized in that** that the flow rate of the feeding is increased linearly with the duration of the feeding.

2. Process according to claim 1, wherein the process is a fed batch process.

3. Process according to claim 1 or 2, wherein the medium used when the feeding occurs is different from the starting medium and wherein the starting medium has a lower concentration of glucose and glutamine than the medium used for the feeding.

4. Process according to claim 3, wherein a feeding concentrate is used for the feeding.

5. Process according to any of claims 1 to 4, wherein the feeding does not commence at the same time as inoculation.

6. Process according to any of claims 1 to 5, wherein the rates of consumption of the nutrients are determined prior to culturing of the cells.

7. Process according to claim 6, wherein the determination of the rates of consumption is performed in a batch process.

8. Process according to any of claims 1 to 7, wherein the consumption rates of the nutrients are not determined during culturing.

9. Process according to any of claims 1 to 8, wherein the cells are mammalian cells.

10. Process according to claim 9, wherein the mammalian cells are primate or rodent cells.

11. Process according to claim 9 or 10, wherein the cells are CHO cells.

12. Process according to any of claims 1 to 11, wherein the host cells are cultured over a period of more than 6 days.

13. Process according to any of claims 1 to 12, wherein the cell density is $\geq 10^7$ cells per ml of culture medium upon termination of the culturing.

14. Process according to any of claims 1 to 13, resulting in a concentration of recombinant protein of ≥60 mg/l culture medium.

15. Process according to any of claims 1 to 14, wherein the recombinant protein is a glycosylated protein.

16. Process according to claim 15, wherein the recombinant protein has a high degree of sialylation.

17. Process according to claim 15 or 16, wherein the recombinant protein is erythropoietin.


**Revendications**

1. Procédé de fabrication de protéines recombinantes dans une ligne de cellules hôtes eucaryotes, la culture des cellules hôte s'effectuant sous alimentation continue en produits alimentaires,
**caractérisé en ce que** la vitesse d'écoulement de l'alimentation est augmentée en dépendance linéaire de la durée de l'alimentation.

2. Procédé selon la revendication 1, pour lequel il s'agit d'un procédé fed-batch.

3. Procédé selon la revendication 1 ou 2, dans lequel l'alimentation utilise un autre milieu que le milieu de départ, le milieu de départ présentant une concentration inférieure en glucose et glutamine que le milieu utilisé pour l'alimentation.

4. Procédé selon la revendication 3, dans lequel un concentré alimentaire est utilisé pour l'alimentation.

5. Procédé selon l'une quelconque des revendications 1 à 4, dans lequel l'alimentation ne débute pas simultanément avec l'inoculation.

6. Procédé selon l'une quelconque des revendications 1 à 5, dans lequel les taux de consommation des produits alimentaires sont déterminés avant la culture.

7. Procédé selon la revendication 6, dans lequel la détermination des taux de consommation s'effectue par un procédé de traitement par lot.

8. Procédé selon l'une quelconque des revendications 1 à 7, dans lequel les taux de consommation des produits alimentaires ne sont pas déterminés pendant la culture.

9. Procédé selon l'une quelconque des revendications 1 à 8, dans lequel les cellules sont des cellules de mammifères.

10. Procédé selon la revendication 9, dans lequel les cellules de mammifères sont des cellules de primates ou de rongeurs.

11. Procédé selon la revendication 9 ou 10, dans lequel les cellules sont des cellules CHO.

12. Procédé selon l'une quelconque des revendications 1 à 11, dans lequel les cellules hôte sont cultivées pendant une période supérieure à six jours.

13. Procédé selon l'une quelconque des revendications 1 à 12, dans lequel à la fin de la culture, on atteint une densité de cellules $\geq 10^7$ cellules par ml de milieu de culture.

14. Procédé selon l'une quelconque des revendications 1 à 13, dans lequel on atteint une densité de protéines recombinantes $\geq$ à 60 mg/l de milieu de culture.

15. Procédé selon l'une quelconque des revendications 1 à 14, dans lequel la protéine recombinante est une protéine glycosylée.

**16.** Procédé selon la revendication 15, dans lequel la protéine recombinante présente un haut degré de sialysation.

**17.** Procédé selon la revendication 15 ou 16, dans lequel la protéine recombinante est de l'érythropoiétine.

Abb. 1 a)

Abb. 1 b)

Abb. 2

Zeit (d)

Abb. 3

Abb. 4

Abb. 5

## IEF/Western

| STD | 4.42 | 3.41 | 2.63 | 1.81 | 1.81 |
| BRP | E1 | E1 | E1 | E1 | A |

Abb. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6180401 B
- US 20020099183 A1
- EP 1036179 A
- WO 9733973 A
- US 5912113 A
- WO 9109955 A
- WO 9309222 A
- EP 1037921 A
- EP 0830376 A
- EP 0228452 A
- WO 03045995 A
- WO 9928346 A
- WO 0345995 A

**In der Beschreibung aufgeführte Nicht-Patentliteratur**

- **HASSELL et al.** *Applied Biochemistry and Biotechnology,* 1991, vol. 30, 29-41
- **LJUNGGREN ; HÄGGSTRÖM.** *Biotechnology and Bioengineering,* 1994, vol. 44, 808-818
- **ZHOU et al.** *Biotechnology and Bioengineering,* 1995, vol. 46, 579-587
- **XIE ; WANG.** *Biotechnology and Bioengineering,* 1994, vol. 43, 1175-1189
- *Biotechnology and Bioengineering,* 1996, vol. 51, 725-729
- *European Pharmacopoiea,* 2002, 73-75
- **URLAUB ; CHASIN.** *PNAS,* 1980, vol. 77, 4216-4220
- *European Pharmacopoiea,* 2002, vol. 01/2002, 1316